(19) **Europäisches Patentamt**
**European Patent Office**
**Office européen des brevets**

(11) **EP 4 606 361 A1**

(12) **EUROPEAN PATENT APPLICATION**

(43) Date of publication:
**27.08.2025  Bulletin 2025/35**

(21) Application number: **24185078.3**

(22) Date of filing: **27.06.2024**

(51) International Patent Classification (IPC):
***A61F 13/53*** *(2006.01)*   ***A61F 13/42*** *(2006.01)*
***A61L 15/28*** *(2006.01)*   ***A61L 15/60*** *(2006.01)*

(52) Cooperative Patent Classification (CPC):
**A61F 13/53; A61F 13/42; A61L 15/60;**
A61F 2013/422; A61F 2013/429;
A61F 2013/530007; A61F 2013/530481;
A61F 2013/530795

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **20.02.2024  EP 24158653**
**04.04.2024  EP 24168436**

(71) Applicants:
• **Ontex BV**
**9255 Buggenhout (BE)**

• **Ontex Group NV**
**9320 Erembodegem (BE)**

(72) Inventors:
• **Idelson, Alissa**
**53359 Rheinbach (DE)**
• **Reuter, Agnes Dominika**
**53359 Rheinbach (DE)**
• **Lambertz, Christina**
**56566 Neuwied (DE)**

(74) Representative: **Saurat, Thibault**
**Ontex BV**
**Legal Department**
**Korte Keppestraat 21**
**9320 Erembodegem (BE)**

(54) **ABSORBENT ARTICLE WITH IMPROVED ABSORBENT CORE**

(57)    An absorbent article (1) comprising:
- a liquid permeable topsheet (2),
- a liquid impermeable backsheet (3), and
- an absorbent core (4) positioned between said topsheet (2) and backsheet (3),
wherein the absorbent core (4) comprises an absorbent material (5), said absorbent material comprising a mixture, or blend, of cellulose fibers (5a) and superabsorbent material (5b), **characterized in that** the superabsorbent material comprises biodegradable superabsorbent material (bioSAP) that has a first color in dry state and a second color in wet state, and **in that** first color and the second color has a CIELab color space difference ($\Delta E$) of at least 2 as measured according to the test method herein.

**FIG. 1**

EP 4 606 361 A1

**Description**

**TECHNICAL FIELD**

The disclosure relates to absorbent articles such as disposable absorbent articles, preferably selected from the group consisting of diapers (whether for baby or adults), pants (whether for baby or adults), pantiliners, briefs, sanitary napkins, and combinations thereof.

**BACKGROUND**

Disposable absorbent garments, or disposable absorbent articles, such as infant diapers or training pants, adult incontinence products and other such products typically were constructed with a liquid impervious outer backsheet, a liquid pervious body-contacting topsheet, and an absorbent core sandwiched between said topsheet and backsheet. Much effort has been made to find cost-effective materials for absorbent cores that display favourable liquid absorbency and retention. Superabsorbent materials in the form of granules, beads, fibers, buits of film, globules, *etc.,* have been favoured for such purposes. Such superabsorbent materials generally (also referred to as superabsorbent polymer particles or SAP) are polymeric gelling materials that are capable of absorbing and retaining large quantities of liquid, such as water and body wastes, relative to their own weight, even under moderate pressure. The superabsorbent material generally is a water-insoluble but water-swellable polymeric substance capable of absorbing water in an amount which is at least ten times the weight of the substance in its dry form. A superabsorbent material comprising a polymer is hereinafter referred to as a superabsorbent polymer or "SAP". In one type of SAP, the particles or fibers may be described chemically as having a backbone of natural or synthetic polymers with hydrophilic groups or polymers containing hydrophilic groups being chemically bonded to the back bone or in intimate admixture therewith. Included in this class of materials are such modified polymers as sodium neutralized cross-linked polyacrylates and polysaccharides including, for example, cellulose and starch and regenerated cellulose which are modified to be carboxylated, phosphonoalkylated, sulphoxylated or phosphorylated, causing the SAP to be highly hydrophilic. Such modified polymers may also be cross-linked to reduce their water-solubility.

There has been a trend in the recent years to incorporate biodegradable or biocompatible superabsorbent polymer more commonly called "bioSAP". Indeed synthetic SAP present issues such as sustainability concerns surrounding the production processes of petroleum-based polymers as well as toxicity concerns stemming from acrylamide from such products and finally a lack of biodegradability of the SAP results in poor end-of-life properties of these products, resulting in the need for disposal e.g. incineration or landfilling. Hence there has been a trend for using more sustainable and biodegradable or biocompatible superabsorbent polymer in absorbent articles.

Documents such as US20230058841, EP4158049, WO2019/118987, US20170224540 or WO2017/061166, to name a few publications, all describe biodegradable high-performance biodegradable or biocompatible absorbent polymers.

However, absorbent articles comprising such biodegradable or biocompatible superabsorbent materials, hence comprising less synthetic, or petroleum-derived, chemical compounds, tend to be perceived by end-consumers as less effective in terms of absorbency and rewet capacities than article comprising conventional SAP. There is hence a need to reassure end-consumers and show that these more environmental-friendly articles are just as effective as conventional articles at least in terms of absorbency and rewet.

**SUMMARY**

The object of the present invention pertains to an absorbent article comprising a liquid permeable topsheet, a liquid impermeable backsheet, and an absorbent core positioned between said topsheet and backsheet, wherein the absorbent core comprises an absorbent material, said absorbent material comprising a mixture, or blend, of cellulose fibers and superabsorbent polymers, preferably said absorbent material is contained within at least one core wrap substrate enclosing said absorbent material therein, **characterized in that** the superabsorbent material comprises biodegradable superabsorbent material (bioSAP) that has a first color in dry state and a second color in wet state, and **in that** first color and the second color has a CIELab color space difference ($\Delta E$) of at least 2, preferably of at least 5, more preferably of at least 10, even more preferably of at least 20 as measured according to the test method herein. In other words, the biodegradable superabsorbent material (bioSAP) has a color difference ($\Delta E^*ab$ or $\Delta E$) greater than 2, preferably greater than 5, more preferably greater than 10, even more preferably of greater than 20 with respect to a dry-state and to a wet-state. A wet-state refers to the condition in which the bioSAP in the absorbent article has been saturated with exudates and/or liquid. Typically wherein at least 10ml, preferably at least 20ml, even more preferably at least 50ml, most preferably from 60ml to 800ml, of exudate and/or liquid are contained in the absorbent article, a wet-state encompasses at least partially wetted. Said wet-state of the bioSAP also encompasses states of the bioSAP where it is at least partially swelled-up. Preferably, the first color and the second color has a C.I.E. L*ab color space difference ($\Delta$E) greater than 2 as measured according to the test method herein, preferably, said color space difference ($\Delta$E) is greater than 5, more preferably said color space difference ($\Delta$E) is greater than 10, more preferably said color space difference ($\Delta$E) is greater than 15, even more preferably said color space difference ($\Delta$E) is greater than 20. The greater the color space difference is the more visible the difference between a dry-state and a wet-state is and hence indicative of absorbency. A color space difference ($\Delta$E) of 2 can be detected with a good eyesight, a greater color space difference can be detected more easily and faster and with less uncertainty.

The color difference for a first color ($L_1^*$, $a_1^*$, $b_1^*$) and a second color ($L_2^*$, $a_2^*$, $b_2^*$), is calculated according to the following formula: $\Delta E = \sqrt{\Delta L^2 + \Delta a^2 + \Delta b^2}$ wherein: $\Delta L = L_1^* - L_2^*$; $\Delta a = a_1^* - a_2^*$; and $\Delta b = b_1^* - b_2^*$, with references 1 and 2 being the dry-state and the wet-state or at least partially wet-state.

By using a superabsorbent polymer that is biodegradable and that enables the absorbent article to have a C.I.E L* score that is different between a dry-state and a wet-state, the end-consumer can see that a significant enough change and deduce an absorbency capacity for the more sustainable absorbent article. In other words, the dry-state, or first state, is visually different compared to the wet-state, or second state, and since the second wet-state is visually discernable from the first dry-state, the caregiver can deduce an efficiency in the environmental-friendly articles in terms of both absorption and rewet thereby corresponding to an effective way to communicate this information since it exploits visual features, i.e. the coloring. In other words, the object of the present disclosure pertains to an absorbent article comprising a liquid permeable topsheet, a liquid impermeable backsheet, and an absorbent core positioned between said topsheet and backsheet, wherein the absorbent core comprises an absorbent material, said absorbent material comprising a mixture, or blend, of cellulose fibers and superabsorbent polymers, preferably said absorbent material is contained within at least one core wrap substrate enclosing said absorbent material therein, **characterized in that** the superabsorbent material comprises biodegradable superabsorbent material (bioSAP) wherein the absorbent article has a first color in dry state and a second color in wet state, and **in that** first color and the second color has a CIELab color space difference ($\Delta$E) of at least 2, preferably of at least 5, more preferably of at least 10, even more preferably of at least 20 as measured according to the test method herein.

According to an embodiment, the cellulose fibers are selected from unbleached pulp or totally chlorine free (TCF) or elemental chlorine free (ECF) cellulose fibers or any combination thereof.

According to an embodiment, the biodegradable superabsorbent material bioSAP is opaque, or semi-opaque, in a dry-state and translucent in a wet-state. For example, the biodegradable superabsorbent material has a $\Delta$L*, *i.e.* a difference of L* value, greater than 5 between a dry-state and a wet-state, preferably greater than 10. In other words, the biodegradable superabsorbent material bioSAP is more translucent in a wet-state than in a dry-state. For example, between a dry-state and a wet-state the biodegradable superabsorbent material bioSAP can vary from L* = 25 to 99, a* = 5 to 45 and b* = 20 to 60 with a $\Delta$L* of at least 10. Preferably, in a dry state the biodegradable superabsorbent material bioSAP has a L* value comprised between 35 and 85, preferably between 38 and 65 whereas in a wet state the biodegradable superabsorbent material bioSAP has a greater L* value, preferably comprised between 45 and 99, preferably between 60 and 99.

According to an embodiment, the $\Delta$L*, meaning the L* value difference of the biodegradable superabsorbent material between the first color in dry state and a second color in wet state bioSAP, is greater than 5, preferably greater than 10.

According to an embodiment, the $\Delta$LT, meaning the light transmission difference of the biodegradable superabsorbent material between the first color in dry state and a second color in wet state bioSAP, is greater than 5, preferably greater than 10.

According to an embodiment, the biodegradable superabsorbent material comprises carbohydrate fermentation-derived polymers.

According to an embodiment, the biodegradable superabsorbent material comprise polysaccharides-derived polymers, preferably having crosslinked polysaccharides.

According to an embodiment, the color space difference $\Delta$E, meaning the color difference of the biodegradable superabsorbent material between the first color in dry state and a second color in wet state bioSAP, is greater than 10, preferably greater than 20.

According to an embodiment, the absorbent core comprises a first and a second layer, said first and second layer containing different grade and/or concentration of biodegradable superabsorbent material.

According to an embodiment, said first layer contains carbohydrate fermentation-derived polymers and second layer contains polysaccharides-derived polymers.

According to an embodiment, said first layer is arranged proximal to the topsheet and said second layer proximal to the backsheet.

According to an embodiment, both first layer and second layer contains carbohydrate fermentation-derived polymers and polysaccharides-derived polymers.

According to an embodiment, the color space difference ΔE between the dry-state and the wet-state is greater than 10, preferably greater than 20.

According to an embodiment, the color space difference ΔE between a dry-state and a wet-state is greater for the first layer than for the second layer.

According to an embodiment, the cellulose fibers are comprised at a level of at least 20%wt, preferably from 25% wt to 40%wt, even more preferably from 29%wt to 37%wt, by total weight of the absorbent material.

According to an embodiment, the absorbent material is wrapped in a core wrap substrate, said core wrap substrate comprising upper and lower layers that are joined together by one or more adhesives and/or by one or more mechanical bonds selected from the group consisting of ultrasonic bonds, thermal bonds, pressure bonds, and combinations thereof, preferably said layers are joined together at one or more attachment zones to form one or more channels, preferably a single channel, substantially free of absorbent material.

According to an embodiment, an acquisition distribution layer is positioned between the topsheet and the core wrap, and wherein the majority of the surface of said acquisition distribution layer is in direct contact at least with said core wrap substrate.

According to an embodiment, the cellulose fibers are comprised at a level of at least 20%wt, preferably from 25% wt to 40%wt, even more preferably from 29%wt to 37%wt, by total weight of the absorbent material.

According to an embodiment, the core wrap substrate comprises upper and lower layers that are joined together by one or more adhesives; and/or by one or more mechanical bonds selected from the group consisting of ultrasonic bonds, thermal bonds, pressure bonds, and combinations thereof; and preferably wherein said layers are joined together at one or more attachment zones to form one or more channels, preferably a single channel, substantially free of absorbent material, and preferably wherein the channel(s) have a shape such to form at least one, preferably at least two, typically only two, clusters of absorbent material circumscribed by said attachment zones and preferably wherein the at least two clusters are spaced apart along a dimension parallel to the longitudinal axis (y), and even more preferably wherein the two or more clusters are connected by one or more attachment zones bridging between said clusters and extending substantially along said longitudinal axis (y).

According to an embodiment, the upper layer is joined to the bottom layer at one or more bonding points positioned inboard of a perimeter of the absorbent core, and preferably wherein said bonding points have an aspect ratio of less than 3, preferably of from 1 to 2, and more preferably having a shape selected from the group consisting of circular, elliptical, line-form, star-shaped, polygonal, and combinations thereof.

According to an embodiment, the article further comprising an acquisition distribution layer positioned between the topsheet and the core wrap, and wherein the majority of the surface of said acquisition distribution layer is in direct contact at least with said core wrap substrate; and wherein the acquisition distribution layer comprises, preferably consists of, a nonwoven selected from the group consisting of spunbond nonwoven, meltblown nonwoven, carded thermobonded nonwoven, and combinations thereof.

The present disclosure also pertains to the use of biodegradable superabsorbent polymer as described herein in an absorbent article to provide a change of color between a dry-state and a at least partially wet-state or wet-state preferably when viewing said absorbent article from above or top-view or from below or bottom-view, preferably to provide an indication of the state of said absorbent article selected from a dry-state, a partially wet-state or wet-state.

The present disclosure also pertains to the use of biodegradable superabsorbent polymer that has a first color in dry state and a second color in wet state said first color and said second color having a CIELab color space difference (ΔE) of at least 2 as measured according to the test method herein, in an absorbent article to provide a change of color between a dry-state and a at least partially wet-state or wet-state preferably when viewing said absorbent article from above or top-view or from below or bottom-view, preferably to provide an indication of the state of said absorbent article selected from a dry-state, a partially wet-state or wet-state.

The present disclosure also pertains to the use of biodegradable superabsorbent polymer (bioSAP) having a ΔE, *i.e.* a difference of E value, greater than 10 between a dry-state and a wet-state in an absorbent article to provide a change of color between a dry-state and a at least partially wet-state, preferably to provide an indication of the state of said absorbent article selected from a dry-state, a partially wet-state or wet-state.

The present disclosure also pertains to the use of biodegradable superabsorbent polymer (bioSAP) comprising carbohydrate fermentation-derived polymers and/or polysaccharides-derived polymers in an absorbent article to provide a change of color between a dry-state and a at least partially wet-state, preferably to provide an indication of the state of said absorbent article selected from a dry-state, a partially wet-state or wet-state.

The present disclosure also pertains to the use of biodegradable superabsorbent polymer in an absorbent article to measure the C.I.E L* scores of said absorbent article in a dry-state and a at least partially wetted state to provide an indication of the state of said absorbent article selected from a dry-state, a partially wet-state or wet-state.

For example, conventional disposable absorbent articles can comprise a wetness indicator, said wetness indicators being visible from the exterior of the article and having the capacity to change appearance when contacted with body exudates, in particular urine. Usually wetness indicators are in the form of a hot-melt adhesive including one or more polymers to provide cohesive strength, a resin or analogous material (tackifier) to provide adhesive strength; and optional waxes, plasticizers or other materials to modify viscosity, and/or other additives including, but not limited to, antioxidants or other stabilizers these components comprises several chemical products and typically several petroleum-based polymers which further present issues such as sustainability. The use of biodegradable superabsorbent polymer in an absorbent article to measure the C.I.E L* scores of said absorbent article in a dry-state and a at least partially wetted state to detect a wet and/or dry-state of absorbent article remove the need for conventional wetness indicators thereby lowering the presence of petroleum derived polymers.

**BRIEF DESCRIPTION OF THE FIGURES**

FIG. 1 illustrates a schematic cross section of an absorbent article according to the present disclosure.
FIG. 2 shows a view in perspective of an absorbent article according to the present disclosure.
FIG. 3 Illustrates a cross-section of an absorbent article according to the present disclosure.
FIG. 4A-C Illustrates absorbent articles according to embodiments according to the present disclosure.
FIF. 5 Illustrates a top view of an absorbent article according to the present disclosure.
FIG. 6 shows an illustration of the CIELAB color space.

**DETAILED DESCRIPTION**

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0,1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent or % wt.), here and throughout the description unless otherwise defined, refers to the relative weight of the respective component based on the overall weight of the formulation.

The terms "biodegradable superabsorbent material" or "bioSAP" or "bipolar superabsorbent material" or "biocompostable superabsorbent material" or "superabsorbent material with a bio-based content" are equivalent terms and means a superabsorbent material that has the property to biodegrade, meaning that this superabsorbent material is capable of being broken down into innocuous products by the action of living elements such as microorganisms. Biodegradation, e.g., based on carbon to carbon dioxide conversion for solid materials (Sturm test), may be performed according to several methods such as ASTM D5210,ASTM D5988, ASTM D5511, ASTM D5864, ASTM D5271 and Iso 14593. Composability can be determined ASTM D6400, ISO 16929, 15014855. In addition, the term "biodegradable" can also mean a carbon-based material can be bacterially and/or enzymatically decomposed under conditions that are typically present in landfills. Such conditions usually include deep burial so that anaerobic bacterial and/or enzymatic activity typically will be the primary mode of decomposition. Such conditions typically do not include substantial non-bacterial hydrolysis but hydrolysis may occur to some extent. Typically, the biodegradable material is converted primarily, substantially, essentially or completely to ultimate decomposition products such as carbon dioxide, water and inorganic residue. The time needed for such full decomposition is usually substantial but bacterial and/or enzymatic decomposition will begin shortly after burial. "Bio-based content" refers to the amount of carbon from a renewable resource in a material as a percent of the mass of the total organic carbon in the material, as determined by ASTM D6866-10, method B. In order to apply the methodology of ASTM D6866-10, method B, to determine the bio-based content of any absorbent article or component thereof.

The terms "C.I. E. L*a*b*" or "C. I. E. L*ab" refer to a color scale utilizes measures of lightness (L*), redness-greenness (a*), and yellowness-blueness (b*) to characterize colors. "C.I.E. L*score" or "C.I.E. L*a*b* values" are equivalent terms and corresponds to the values measured according to said C.I.E. L*a*b* scale. ΔE corresponds to the difference of "C.I.E. L*a*b* values between two states and/or regions and thereby corresponds to the difference of color between two states and/or regions, or more simply, ΔE corresponds to the color difference or the CIELab color space difference between two states and/or regions.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

"Laminate" refers to elements being attached together in a layered arrangement.

The term "spunbond fibers (or layer(s) or nonwovens)" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 μm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc.

The recitation of numerical ranges by endpoints includes all numbers and fractions subsumed within that range, as well as the recited endpoints unless otherwise stated.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement. Carded thermobonded nonwoven thus refers to a carded nonwoven wherein the bonding is achieved by use of heat.

The term "top sheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

"Adhesive" typically means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "back sheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

As used herein, the term "transverse" or "lateral" refers to a line, axis, or direction which lies within the plane of the absorbent article and is generally perpendicular to the longitudinal direction.

"Thickness or caliper" herein are used interchangeably, and refer to the caliper typically measured as follows: at 0,5 kPa and at least five measurements are averaged. A typical testing device is a Thwing Albert ProGage system. The diameter of the foot is between 50 mm to 60 mm. The dwell time is 2 seconds for each measurement. The sample is to be stored at 23 + 2°C and at 50 + 2% relative humidity for 24 hours with no compression, then subjected to the fabric thickness measurement. The preference is to make measurements on the base substrate before modification, however, if this material is not available an alternative method can be used. For a structured substrate, the thickness of the first regions in between the second regions (displaced fiber regions) can be determined by using a electronic thickness gauge (for instance available from McMaster-Carr catalog as Mitutoyo No 547- 500). These electronic thickness gauges can have the tips changed to measure very small areas. For example, a blade shaped tip can be used that is 6,6mm long and 1mm wide. Flat round tips can also be inserted that measure area down below 1,5mm in diameter. For measuring on the structured substrate, these tips are to be inserted between the structured regions to measure the as-produced fabric thickness. The pressure used in the measurement technique cannot be carefully controlled using this technique, with the applied pressure being generally higher than 0,5kPa.

"Dry-state" or "dry state" refer to the condition in which an absorbent article has absorbed little to no exudates and/or liquid, preferably a condition in which the absorbent article has not been in contact with any exudate and/or liquid. The absorbent article in a dry state retains its original dry weight and show no visible signs of saturation or leakage.

"Wet-state" or "wet state" refer to the condition in which an absorbent article has been saturated with exudates and/or liquid. In other words, the absorbent article has absorbed a significant amount of liquid, close to or at its maximum capacity, and may show visible signs of saturation or leakage. Typically wherein at least 30ml, preferably at least 40ml, even more preferably at least 50ml, most preferably from 60ml to 800ml, of exudate and/or liquid are contained in the absorbent article. For example, an absorbent article might be defined in a wet-state when it has absorbed 50% or more of its total or maximum absorption capacity, meaning for example if an absorbent article has an absorption capacity of 400 mL, it will be considered in a wet-state if it has absorbed 200 mL or more of exudate and in a partially wet-state if it has absorbed less. An absorbent article might also be considered in a wet-state if its weight has increased by a certain percentage, such as 20% or 30% or more, due to liquid absorption. For instance, if an absorbent article's weight is increased by 50 grams after liquid absorption, it could be considered in a wet-state. Within the scope of the present disclosure, "wet-state" encompasses partially wetted or at least partially wet-state. For the sake of precision, "partially wetted" or "partially wet-state" or "partially wet state" refer to the condition in between a dry-state and a wet state in which an absorbent article has been in contact with exudates and/or liquid but is not yet saturated. In other words said absorbent article has absorbed some exudates and/or liquid but has not yet reached the threshold that qualifies it as in a wet-state or simply put the absorbent article has absorbed some exudate and/or liquid but has not reached a given absorption capacity, it shows signs of dampness or slight weight increase, falling between the dry and wet states. According to the present disclosure, the biodegradable superabsorbent particle (bioSAP) and thereby the absorbent article show a change of color in a partially wet-state and in a wet-state compared to a dry-state. Typically wherein at least 5 ml, even more preferably at least 10 ml or 30 mL, of exudate and/or liquid are contained in the absorbent article. For example, an absorbent article might be defined in a partially wet-state when it has absorbed between 5% and 50% of its total or maximum absorption capacity. An absorbent article might be considered in a partially wet-state if its weight has increased by a certain percentage, such as between 5% or 20%, due to liquid absorption. For instance, if an absorbent article's weight is increased by 10 grams after liquid absorption, it could be considered in a partially wet-state. The term "at least partially wet-state" encompasses both "partially wet-state" and "wet-state".

As used herein, the term "cellulosic" or "cellulose" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

By "substantially", it is meant at least the majority of the structure referred to.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "consisting essentially of' does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

As used herein, the "body-facing" or "bodyside" or "skin-facing" surface means that surface of the article or component which is intended to be disposed toward or placed adjacent to the body (e.g. the face) of the wearer during ordinary use, while the "outward", "outward-facing" surface is on the opposite side, and is intended to be disposed to face away from the wearer's body during ordinary use.

"Spunlaced" as used herein refers to nonwoven fabrics or materials that are made by hydroentangling webs of fibers (and/or fibers) with high energy water jets for example as basically described in Evans et al. US Patent No. 3,485,706. The webs may be made of a variety of fibers such as polyester, rayon, cellulose (cotton and wood pulp), acrylic, and other fibers as well as some blends of fibers. The fabrics may be further modified to include antistatic and antimicrobial properties, etc. by incorporation of appropriate additive materials into the fiber or fiber webs.

"Wetlaid" as used herein means nonwovens obtained by a process similar to paper manufacturing. The difference lies in the amount of synthetic fibres present in a wetlaid nonwoven. A dilute slurry of water and fibres is deposited on a moving wire screen, where the water is drained and the fibres form a web. The web is further dewatered by pressing between rollers and dried. Impregnation with binders is often included in a later stage of the process.

"Airlaid" as used herein means a process wherein fibres, which are typcially relatively short, are fed into a forming head by an airstream. The forming head assures a homogeneous mix of all fibres. By air again, a controlled part of the fibre mix leaves the forming head and is deposited on a moving belt, where a randomly oriented web is formed. Compared with carded webs, airlaid webs have a lower density, a greater softness and an absence of laminar structure.

The term "grades" used in "superabsorbent polymer grades" typically refers to the chemichal properties e.g. monomer and charge ratio; and physicochemical properties e.g. network structure, degree of cross-linking, post treatment and particle size distribution, of the superabsorbent polymer. Typically, these properties (or grades) result in unique performance parameters such as AUL, absorbption speed (or Vortex), CRC and the like.

Embodiments of the articles and processes according to the disclosure will now be described. It is understood that technical features described in one or more embodiments maybe combined with one or more other embodiments without departing from the intention of the disclosure and without generalization therefrom.

ABSORBENT ARTICLE

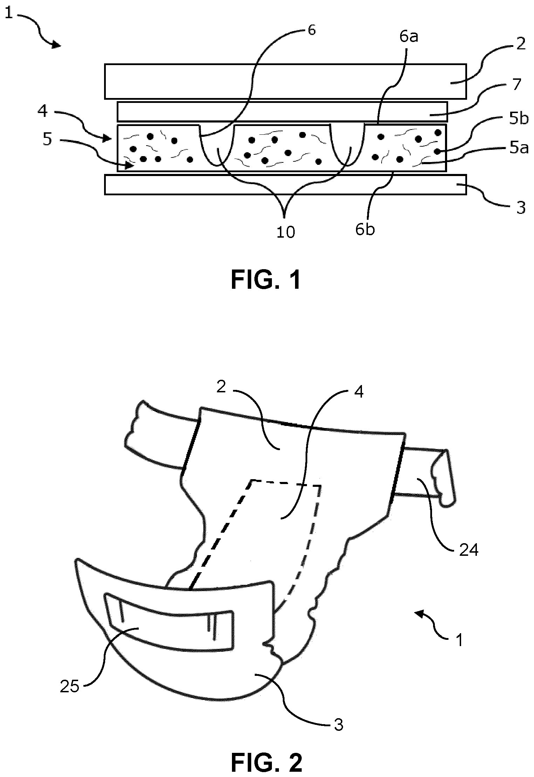

As exemplified in Fig. 1, absorbent articles (1) as described herein comprise: a liquid permeable topsheet (2), a liquid impermeable backsheet (3), and an absorbent core (4) positioned between said topsheet (2) and backsheet (3), wherein the absorbent core (4) comprises an absorbent material (5), said absorbent material comprising cellulose fibers (5a) and/or superabsorbent polymers (5b). The absorbent material (5) can be contained within at least one core wrap substrate (6) enclosing said absorbent material (5) therein. The core wrap substrate (6) may be a single sheet that is folded onto itself to contain the absorbent material (5) therein or may comprise two distinct sheets that are bonded to each other at least at the peripheral edges thereof to sandwich the absorbent material (5) therein. Several core wrap constructions are possible such as G-fold, C-fold or sandwich wraps. The core wrap substrates (6) herein are typically composed of a nonwoven layer or nonwoven laminates comprising spunbond nonwoven, meltblown nonwoven and combinations thereof or tissue. Typically the basis weight of the core wrap substrate is from 5 gsm to 50 gsm, preferably from 6 gsm to 35 gsm, more preferably from 8 gsm to 30 gsm. The absorbent material (5) may be contained within the absorbent article (1) by the topsheet and backsheet (3) only without the presence of a core wrap substrate (6). Since the object pertains to environmentally friendly absorbent articles, according to the present disclosure, the core wrap substrate 6 can be made of environmentally friendly material, preferably biodegradable and/or compostable material, selected from and not limited to polylactic acid (PLA), viscose, polyhydroxyalcanoates (PHA), bio-derived polyethylene, (bioPE), poly-3-hydroxybutyrate (PHB) or polycaprolactone (PCL).

As illustrated in FIG. 2, the absorbent article 1 can be a diaper comprising elastic ears 24 and a landing zone 25 comprising attachment means such as hooks and loops or adhesive to attach the elastic ears 24 to the landing zone 25. The absorbent article 1 can also be a pant, such as a training pants or an incontinence pants where the sides of the front and back panels in facing relationship are seamed together thereby forming a wearable absorbent article. The absorbent article 1 can also be a feminine hygiene product such as a sanitary napkin or a pantiliner or an incontinence pad.

The absorbent article 1 comprises absorbent material (5) comprising cellulose fibers (5a) and/or superabsorbent polymers (5b). Superabsorbent polymers, superabsorbent polymers particles, or SAPs refer to water-swellable, water-insoluble organic or inorganic materials capable of absorbing at least about 10 times their weight, or at least about 15 times their weight, or at least about 25 times their weight in an aqueous solution containing 0,9 weight percent sodium chloride. In absorbent articles, such as diapers, incontinent diapers, etc., the particle size is typically ranging between 100 to 800 $\mu$m, preferably between 300 to 600 $\mu$m, more preferably between 400 to 500 $\mu$m. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0,9 wt % NaCl). The superabsorbent material according to the present disclosure comprises a minima a grade of biodegradable or bipolar or biocompostable or with a bio-based content superabsorbent material. The biodegradable hydrogel-forming polymeric superabsorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose. According to some embodiment, the superabsorbent material can comprise in addition synthetic hydrogel-forming polymers. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present disclosure in an amount up to about 90% by weight.

As explained hereabove, the absorbent material (5) comprises cellulose fibers (5a) and/or superabsorbent polymers (5b). The superabsorbent polymers (5b), or superabsorbent particles, according to the present disclosure is biodegradable or biocompostable or with a bio-based content and will be referenced as bioSAP hereunder.

The biodegradable superabsorbent material (bioSAP) according to the present disclosure comprises a suitable biodegradable superabsorbent base material.

Said biodegradable superabsorbent base material can comprise polysaccharides-derived polymers said material having crosslinked polysaccharides. Example of such crosslinked polysaccharide comprise material such as carboxymethyl cellulose based material crosslinked polycarboxy polysaccharide or carboxyalkylated polysaccharide such as carboxymethyl starch. Other example of polysaccharides include those that have a backbone substituted with ionic (polar) substituents, such as alkylcarboxylate or amine groups. Amine groups occur naturally in some polysaccharides, such as chitosan, while carboxylate groups must be artificially added to the most abundant polysaccharides, cellulose and starch, using carboxy alkyl donating reagents. The most common carboxyalkylaled polysaccharides are carboxymethyl cellulose (CMC) and carboxymethyl starch (CMS). These polysaccharides-derived polymeric material can be used as outright substitutes for conventional polyacrylates SAPs or as grafts or copolymers that may be used in conjunction with conventional polyacrylates SAPs. These materials have a light tan color in a dry-state and are substantially translucent in a wet-state.

Said biodegradable superabsorbent base material can comprise carbohydrate fermentation-derived polymers in addition or alternatively to the polysaccharides-derived polymers. These material include components comprising alcohol such as isopentyl alcohol and 2-methyl-1-butanol, and to a lesser degree contains isobutyl alcohol, n-propyl alcohol, and small amounts of other alcohols, esters and aldehydes. In addition, n-butanol may be derived from the fermentation of acetone/ethanol or from the catalytic condensation of ethanol. The reduced or partially reduced ethers or esters containing either a hydroxyl or amine functionality are used to functionalize oxidized (acidic) starch. These functionalized starches which are carbohydrate fermentation-derived compounds can be used as outright substitutes for conventional polyacrylates SAPs or as grafts or copolymers that may be used in conjunction with conventional polyacrylates SAPs. These materials have a yellow or light-yellow color in a dry-state and are substantially translucent in a wet-state.

According to the present disclosure, it is preferable to use such biodegradable superabsorbent base material as polysaccharides-derived polymers or carbohydrate fermentation-derived polymers base material, tend to have a light tan or light-yellow color and hence can impart the absorbent core with a light tan or light-yellow color. The carbohydrate fermentation-derived polymers tend to have an even more contrasted color, namely a yellowish tan due to the fermentation process and hence can produce a stronger visual difference.

The biodegradable superabsorbent material is such that it enables the absorbent article (1) to have a C.I.E L* score that is different between a first dry-state and a second wet-state. With such biodegradable superabsorbent material, the end-consumer can see a significant enough change in color and deduce an absorbency capacity of the absorbent article. In other words, the dry-state, or first state, is visually different compared to the wet-state, or second state, and since the second wet-state is visually discernable from the first dry-state, the caregiver can deduce an efficiency of the environmental-friendly articles in terms of both absorption and rewet.

In order to assess this, different visual measurements have been carried out as described hereunder:

Visual measurements

Measurements of were carried out using a suitable spectrophotometer, example of such spectrophotometer include a CR300 with program ChromaControl (S) from Konica Minolta with D65 standard illumination, a 2° observer. Visual measurements were carried out at four different positions 13 of measurement of the absorbent article in a dry-state laid flat on a surface, namely prior to any liquid being poured onto the absorbent article. The absorbent article is then applied onto a mannequin (e.g. Maxi or Junior, of either sex), for example for a diaper by catching the tapes and applying them onto the landing zone on the front thereby closing the absorbent article around the mannequin. The mannequin include a liquid distribution system (e.g. a Capability Tester) that can inject liquid, namely a saline solution (e.g. synthetic urine solution or saline solution with 0,9 wt % NaCl) without a coloring dye. The liquid distribution system can inject liquid at different positions (e.g. belly or back) to better mimic the miction points. For example for a Maxi/Maxi Plus diaper 200 mL are injected onto the absorbent article according to the following scheme :

| | Dose 1 | Pause 1 | Dose 2 | Pause 2 | Dose 3 | Pause 3 | Dose 4 | End Pause |
|---|---|---|---|---|---|---|---|---|
| Volume (mL) Time (s) | 75 | 300 | 25 | 300 | 75 | 300 | 25 | 300 |

After the End Pause, the absorbent article is laid flat on a surface and visual measurements were carried out at four different positions 13 of measurement of the absorbent article in a wet-state laid flat on a surface. Of course the volumes can be adjusted, for example injecting only 30 mL or 50 mL or 100 mL in a Maxi/Maxi Plus diaper to have a partially wet-state.

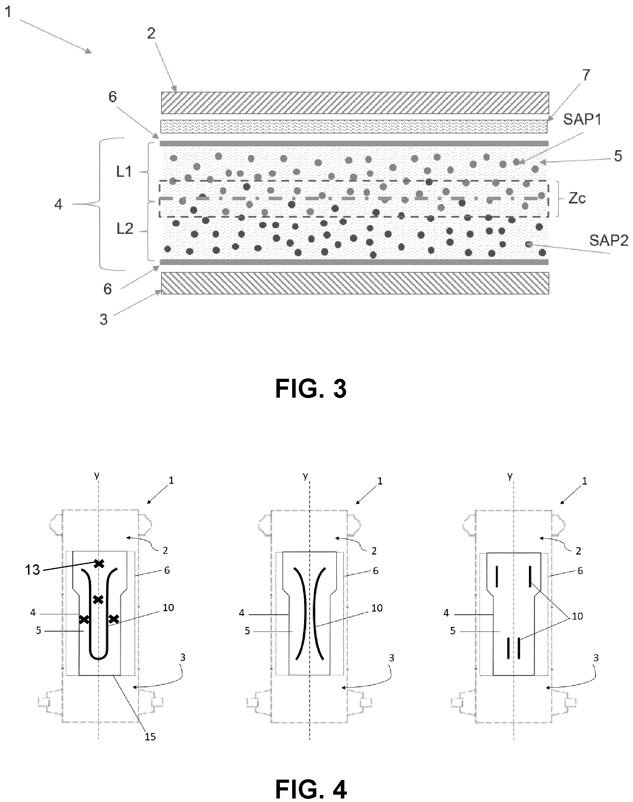

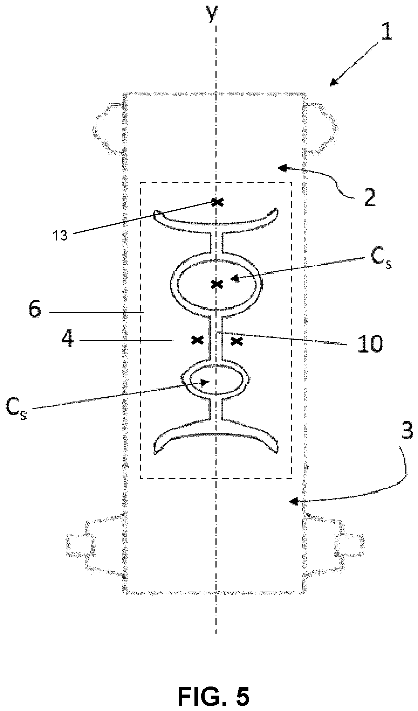

Whatever the sate (wet, dry, partially wet,...), measurements were carried out with the absorbent article being laid flat on a surface, for example a white ceramic tile as a standard backing with values L* 96,0, a* 0,1 and b* 2,85, with the spectrophotometer being placed on top, meaning the spectrophotometer being placed in facing relationship to the topsheet, or body-facing side, of the absorbent article. The four positions 13 of measurement are illustrated by the X in FIG. 4 - left hand drawing with : a first measurement being carried out at the center of the absorbent article with respect to the transversal axis and 5 cm from the edge of the absorbent core (position 1), a second measurement carried out 7 cm from the position 1 (position 2) with respect to the longitudinal axis, a third and fourth measurement carried out 5 cm from position 2 with respect to the longitudinal axis and on each side of the channel as illustrated in FIG. 4. All measurement positions are aligned on the longitudinal axis y-y arranged at the transversal center of the absorbent article. FIG. 5 shows another arrangement of the positions 13 of measurement with four positions 13 of measurement.

The parameters measured with the spectrophotometer are explained hereunder.

Coulour or C.I.E. L*a*b* Measurement:

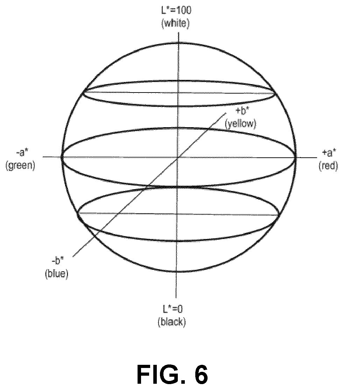

A visual difference between the dry-state and the wet-state may be measured as a difference in color. Color(s) may be characterized by C.I.E. L*, a*, b* values, or C.I.E. L* scores, according to the Color Test Method described herein. The C.I.E. L*, a*, b* color scale utilizes measures of lightness (L*), redness-greenness (a*), and yellowness-blueness (b*) to characterize colors. In other terms, color is determined within the L*a*b* color space, as established by the Commission Internationale de l'Eclairage (CIE) in 1976. The color space is divided into three axes as shown in FIG. 6. L* represents lightness and the axis extends from 0 (black) to 100 (white). The axis a* goes from green to red, where positive values indicate more saturated red, and negative values more saturated green. The b*axis goes from blue to yellow, where positive values represent more saturated yellow and negative values more saturated blue. This color space is well known in industry and is generally referred to as CIE L*a*b* or CIELAB (1976).

To analyse the C.I.E. L*a*b* color values, or C.I.E. L* scores, the selected region around the four positions 13 of measurement were identified and used to visually discern a color change when compared to another state (dry-wet) of the sample.

Using the image analysis software, the average C.I.E. L*, a*, and b* values were measured from selected region around the four positions 13 of measurement. This procedure is repeated on four samples with their C.I.E. L*, a*, and b* values measured and recorded accordingly.

The average L*, a*, and b* values within the selected regions around the four positions 13 of measurement within a first dry-state is calculated and identified as $L_1^*$, $a_1^*$, and $b_1^*$. The average L*, a*, and b* values are then measured for a second wet-state within these same identical regions, and identified as $L_2^*$, $a_2^*$, and $b_2^*$. $\Delta E$, representing the difference of color between two states and/or regions value, is then calculated according to the following equation (1):

$$\Delta E = \sqrt{(L_2^* - L_1^*)^2 + (a_2^* - a_1^*)^2 + (b_2^* - b_1^*)^2} \quad (1)$$

A difference between two colors in the color space CIE L*a*b* is characterised by a Delta E-value ($\Delta E^*ab$). The differences between the colors on the three respective axes are squared in this, following which the differences are summed and the root derived from the sum. Applying this formulae, it is possible to assess the color difference between two states.

This procedure is repeated on four samples with identified replicate regions and $\Delta E$ measured and recorded accordingly.

According to the present disclosure the C.I.E. L* score, representative of a color, between the dry-state and wet-state is different. Meaning for a given, or identical, region of the absorbent article, the C.I.E. L*a*b* values or C.I.E. L* score of the dry-state is different from the C.I.E. L*a*b* values or C.I.E. L* score of the wet-state. Preferably, the $\Delta E$ between the dry and wet-state is greater than 10. More preferably, the $\Delta E$ between the dry and wet-state is greater than 15. Even more preferably, the $\Delta E$ between the dry and wet-state is greater than 20. For example, the $\Delta E$ between the dry and wet-state is comprised between 25 and 100. With such $\Delta E$ or color difference, the care-giver can deduce an efficiency in the environmental-friendly articles in terms of both absorption and rewet. By using such biodegradable superabsorbent material it is possible to see a color difference without resorting to petroleum derived material.

According to an embodiment, the biodegradable superabsorbent material has a $\Delta L^*$, *i.e.* a difference of L* value, greater than 5 between a dry-state and a wet-state, preferably greater than 10.

According to the present disclosure, the topsheet and/or backsheet can have a lower opacity or higher light transmission to better see the color change ($\Delta E$) of the bioSAP. For example, the topsheet and/or backsheet have an opacity that is lesser than the absorbent core.

According to an embodiment, the absorbent article 1 does not comprise any acquisition distribution layer 7, such layer can hinder the care-giver from perceiving a color difference or $\Delta E$ between a dry-state and a wet-state. In other words the absorbent article 1 is free of any acquisition distribution layer 7, or acquisition distribution layer-free, and the absorbent core 4 is arranged directly between the topsheet 2 and backsheet 3, eventually with adhesive in between, the absorbent core 4 comprising absorbent material 5 and eventually core wrap substrates 6.

According to an embodiment, the core wrap substrates 6 comprise an upper core wrap 6a and a lower core wrap 6b. The upper core-wrap 6a and the lower core-wrap 6b can be made of the same material such as SM (spunbond meltblown) laminate, a spunbond or a spunbond laminate e.g. SS), or a carded nonwoven. Preferably, the basis weight of the upper core-wrap 6a is greater than the basis weight of lower core-wrap 6b, the basis weight of upper core wrap is comprised between 15 and 60 gsm, preferably between 18 and 50 gsm, more preferably between 19 and 45 gsm, even more preferably between 20 and 35 gsm whereas the basis weight of the bottom core wrap 6b is comprised between 5 and 15 gsm, preferably between 6 and 12 gsm.

Opacity can be measure after the following method : after a calibration of the instrument per the vendor instructions using the standard white and black tiles provided by the vendor, said spectrophotometer can be set to use the CIE XYZ color space with a D65 standard illumination, a 2° observer, and UV filter to nominal. The material, e.g. topsheet, backsheet and/or absorbent core surface of the test sample can be set over the aperture and ensure that the entire aperture opening is covered by the testing site graphic. Placing the standard white tile directly against the back side of the sample, taking a reading and recording the Y value gives the $Y_{white\ backing}$. Without moving the position of the test sample, removing the standard white tile and replacing it with the black standard tile gives the Y value as $Y_{black\ backing}$.

Opacity is then calculated by applying equation (2):

$$\text{Opacity} = \frac{Y_{black\ backing}}{Y_{white\ backing}} \text{ x } 100 \quad (2)$$

According to an embodiment, the opacity of the topsheet and/or backsheet is lesser than 70%, preferably lesser than 50%, more preferably lesser than 35%.

According to the present disclosure, the biodegradable superabsorbent material bioSAP is at least partially opaque, or semi-opaque, in a dry-state and at least partially translucent, preferably substantially translucent, in a wet-state, opaque meaning preventing, or hindering or impeding light from travelling through whereas translucent meaning allowing light to pass through. For example, the biodegradable superabsorbent material has a light transmission or $\Delta LT$, greater than 5 between a dry-state and a wet-state, preferably greater than 10.

The light transmission test method measures the average amount of light transmitted through specific regions of a patterned fibrous substrate specimen. A calibrated light transmission image is obtained using a flatbed scanner and color management software. The light transmission measurement is based on the CIE L*a*b* color system (CIELAB). The color calibrated image is analyzed using image analysis software.

For example, A flatbed scanner capable of scanning a minimum of 24-bit color at 800 dpi and has manual control of color management (a suitable scanner is an Epson Perfection V750 Pro from Epson America Inc., Long Beach Calif. or equivalent) is used to acquire images. The scanner is interfaced with a computer running color management software (suitable color management software is MonacoEZColor available from X-Rite Grand Rapids, Mich. or equivalent). The scanner is calibrated against a color transparency target and corresponding reference file compliant with ANSI method IT8.7/1-1993 using the color management software to construct a calibrated color profile. The resulting calibrated scanner profile is used to color correct an image from a test specimen within an image analysis program that supports sampling in CIE L*a*b* (a suitable program is Photoshop S4 available from Adobe Systems Inc., San Jose, Calif or equivalent). All testing is performed in a conditioned room maintained at about $23\pm2°$ C. and about $50\pm2\%$ relative humidity. Open the scanner lid and place the specimen, e.g. the bioSAP, against the scanner glass with the outward facing surface facing the glass. Acquire and import a scan of the specimen region within the interior of the frame into the image analysis software at 24-bit color and at 800 dpi in transparency mode. Transparency mode illuminates the specimen from one side with the sensor capturing the image from the opposite side. Assign the calibrated color profile to the image and change the color space mode to L*a*b* Color corresponding to the CIE L*a*b* standard. This produces a color corrected image for analysis

An absorbent article according to the present disclosure referenced herein as Example 1 comprises a topsheet made of 12 gsm polyolefin nonwoven, a backsheet comprising a laminate of a 12 gsm nonwoven and a 14 gsm impermeable film, an absorbent core wrapped in a 8 gsm core wrap comprising a mixture with biodegradable superabsorbent material derived from polysaccharide with a basis weight of 4 gsm and synthetic superabsorbent material with a basis weight of 8,8 gsm, for a total of 12,8 gsm, the absorbent core further comprising cellulosic fluff with a basis weight of 5 gsm. The absorbent core comprises two layers, the top layer comprising the biodegradable superabsorbent material and fluff and the bottom layer comprising the synthetic superabsorbent material and fluff, the channel has a shape as illustrated in FIG. 4 with the measurements being carried out at the four positions 13 of measurement marked by the X (Example 1).

An absorbent article according to the present disclosure referenced herein as Example 2 comprises a topsheet made of 14 gsm polyolefin (e.g. polyethylene, polypropylene) nonwoven, a backsheet comprising a laminate of a 13 gsm nonwoven and a 14 gsm impermeable film (e.g. polyethylene film), an absorbent core wrapped in an 8 gsm core wrap comprising a biodegradable carbohydrate fermentation-derived superabsorbent material with a basis weight of 12,3 gsm and cellulosic fluff with a basis weight of 7 gsm, the channel has a shape as illustrated in FIG. 5 with the measurements being carried out at the four positions 13 of measurement marked by the X (Example 2).

An absorbent article according to the present disclosure referenced herein as Comparative Example comprises a topsheet made of 14 gsm polyolefin nonwoven, a backsheet comprising a laminate of a 13 gsm nonwoven and a 14 gsm impermeable film, an absorbent core wrapped in an 8 gsm core wrap comprising a polyacrylate superabsorbent material, i.e. conventional SAP, with a basis weight of 12,3 gsm and cellulosic fluff with a basis weight of 7 gsm, the channel has a shape as illustrated in FIG. 5 with the measurements being carried out at the four positions 13 of measurement marked by the X (Comparative Example).

The measurement are resumed in the Table hereunder:

| | L* (dry) | L* (wet) | a*(dry) | a*(wet) | b*(dry) | b*(wet) | $\Delta E$ (dry-wet) |
|---|---|---|---|---|---|---|---|
| Comparative Example | | | | | | | 20,9782 |
| Position 1 | 91,806 | 65,725 | -0,224 | -0,294 | 1,484 | -0,709 | 26,1731 |
| Position 2 | 94,636 | 71,805 | -0,265 | -0,121 | 1,834 | -0,866 | 22,9905 |

(continued)

|  | L* (dry) | L* (wet) | a*(dry) | a*(wet) | b*(dry) | b*(wet) | ΔE (dry-wet) |
|---|---|---|---|---|---|---|---|
| Comparative Example | | | | | | | 20,9782 |
| Position 3 | 93,784 | 77,029 | -0,233 | 0,013 | 1,403 | -0,596 | 16,8756 |
| Position 4 | 93,503 | 75,743 | -0,283 | -0,072 | 1,382 | -0,617 | 17,8734 |
| Example 1 | | | | | | | 27,8678 |
| Position 1 | 91,927 | 57,124 | -0,148 | -0,364 | 7,285 | 1,358 | 35,3047 |
| Position 2 | 91,144 | 66,098 | -0,033 | -0,162 | 5,608 | 0,534 | 25,5551 |
| Position 3 | 93,189 | 65,176 | 0,039 | -0,471 | 5,211 | 0,892 | 27,4339 |
| Position 4 | 92,323 | 69,661 | 0,237 | -0,094 | 5,349 | 0,500 | 23,1773 |
| Example 2 | | | | | | | 28,9183 |
| Position 1 | 92,043 | 58,3020 | -0,121 | -0,316 | 1,629 | -0,951 | 33,8401 |
| Position 2 | 94,085 | 65,5850 | -0,146 | -0,268 | 2,242 | -0,937 | 28,6770 |
| Position 3 | 93,359 | 68,2780 | -0,053 | -0,151 | 2,100 | -0,761 | 27,9218 |
| Position 4 | 92,804 | 67,7400 | -0,102 | -0,193 | 2,072 | -0,852 | 25,2341 |
| Position 1 to 4 are taken as illustrated in FIG. 4 or 5 and described hereabove from the top (position 1) to bottom (positions 3 and 4). | | | | | | | |

We can see from the above that absorbent article comprising biodegradable superabsorbent material comprise a greater ΔE between a dry state and a wet state than absorbent article comprising conventional superabsorbent material. Indeed as shown above, absorbent articles according to the example comprising bioSAP comprise an average ΔE between 27 and 29 (27,8678 and 28,9183) whereas an article with conventional SAP comprise an average ΔE of 20,9782. Such visual difference is possible by using superabsorbent material (5b) comprising biodegradable superabsorbent material (bioSAP) that has a first color in dry state and a second color in wet state, and in that first color and the second color has a CIELab color space difference (ΔE) of at least 2. It is apparent with the above, that the biodegradable superabsorbent polymer in an absorbent article can be used to measure the C.I.E L* scores of said absorbent article in a dry-state and a at least partially wetted state to provide an indication of the state of said absorbent article selected from a dry-state, a partially wet-state or wet-state. The difference in C.I.E. L*a*b* values between the positions and between the articles comprising bioSAP can depend on several factors such as the concentration of bioSAP or the nature of the bioSAP (polysaccharide-derived or fermentation-derived).

The present disclosure pertains to more environmentally friendly alternatives to conventional absorbent articles. According to an example, the absorbent article 1 according to the present disclosure comprises totally chlorine free (TCF) or elemental chlorine free (ECF) cellulose fibers (5a). For example, these TCF or ECF cellulose fibers can comprise a Kappa number comprised between 5 and 20 as determined by any standard method such as method ISO 302:2004 or TAPPI Standard Method No. T-214-SU71, where $K \approx c*l$ (with K: Kappa number; c: constant $\approx$ 6,57 (dependent on process and wood); l: lignin content in percent). Such totally chlorine free (TCF) or elemental chlorine free (ECF) cellulose fibers are material which does not utilize chlorine in the whitening process.

According to the present disclosure, the absorbent article 1 comprises unbleached cellulose fibers or unbleached pulp. In accordance with embodiments of the present disclosure, the absorbent core comprises of about 40% to 80% superabsorbent polymer and anywhere from 60% to 20% of the unbleached pulp, preferably between 40% and 25%. For example, some embodiments may utilize only unbleached pulp, while others may utilize unbleached pulp as a percentage of the fiber content. Specifically, in one embodiment, the unbleached pulp content of absorbent core may be as low as 25% of the fiber content and then the remaining 75% of the fiber content would be either a bleached, totally chlorine-free (TCF) or an elemental chlorine-free (ECF) pulp. Unbleached pulp, in accordance with some embodiments, may include a mixture of softwood and hardwood fiber content. Hardwood fibers generally provide much shorter and less coarse fiber with respect to softwood fibers and hence can improve capillarity. For example, in some embodiments the unbleached pulp may have as much as 60% hardwood fibers. Additionally, unbleached pulp has a brown color, because of this brown fiber content in the absorbent core, the end product takes on a light tan color. As noted above, such a tan color in most applications or products is seen as unmarketable for appearance sake. However, because of the unbleached materials, when the product is in use and it is wetted, the fibers appear much darker. Once the fibers are wetted, the surface color at the topsheet or backsheet changes as the fibers come in contact with one another. As a result, the appearance of the core through the backsheet becomes much darker at the point of insult or where the article is wet thereby contrasting more with the bioSAP which in turn is turning more and more light tan. As a result, the diaper can actually give an indication that it has been wetted.

In the current state of the art, it is believed that bleached fibrous material is advantageous both from a performance and from a cosmetic standpoint. Using more environmentally friendly material such as TCF or ECF fibers is beneficial but can further lead end-consumers to perceive these articles as less effective. Hence the usage of biodegradable superabsorbent base material with a C.I.E. L* score that is different between a dry-state and a wet-state is a good way to reassure end-consumers and show that these articles are just as effective. For example, with absorbent articles comprising conventional bleached cellulose and using conventional superabsorbent base material, having a C.I.E. L* score difference, meaning a color change or $\Delta E$, of 5 between the dry and wet states is sufficient to convey a sense of absorbency. For absorbent articles comprising ECF or TCF cellulose fibers, it is preferable to use a biodegradable superabsorbent base material with a C.I.E. L* score difference meaning a color change or $\Delta E$, of at least 2 to effectively convey a sense of absorbency.

The absorbent core (4) can comprise one, two, three or more layers of absorbent material (5). When the absorbent core (4) comprises one layer of absorbent material (5), the absorbent material (5) comprises at least one superabsorbent base material preferably in combination with TCF or ECF cellulose fibers or with conventional cellulose fibers. The absorbent material (5) can comprise two types of superabsorbent base materials, such as polysaccharide-derived and fermentation-derived bioSAP preferably in combination with TCF or ECF cellulose fibers or with conventional cellulose fibers. When there is more than one layer, for example two layers L1,L2 of absorbent layers as illustrated in FIG. 3, said layers L1,L2 can have the same amount or different amounts of absorbent material (5). For example the layers L1, L2 can comprise different amounts of cellulose fibers (5a) and/or biodegradable superabsorbent polymers (5b), different dimensions, different thickness, *etc.* The absorbent core 4 according to the embodiment as illustrated in FIG. 3, comprises the at least one top (or upper) layer (L1) and bottom layer (L2) that are directly stacked one on top of the other to form a contact zone (Zc) where the top layer (L1) directly adjoins to the bottom layer (L2). It is to be understood herein that more than two layers are possible and may be stacked one on top of the other provided that the uppermost layer is in the form of the top layer herein, all other layers may be in the form of bottom layers as described herein. Preferably, the core is free of any additional substrates separating the top and bottom core layers thus allowing some blending of SAP1 and SAP2 at the contact zone which promotes optimal fluid distribution and reduced material usage.

According to the present disclosure, the top and/or bottom layer (L1,L2) comprises biodegradable superabsorbent material (bioSAP) that enables a C.I.E. L* score difference between a dry-state and a wet-state. In other words, at least one layer (L1,L2) comprises biodegradable superabsorbent material (bioSAP) that enables the absorbent article 1 to have a $\Delta E$ greater than 10 between a dry-state and a wet-state.

According to an embodiment, the top layer (L1) of the absorbent core comprises one or more first biodegradable superabsorbent polymer grades (bioSAP1) and the bottom layer (L2) comprises one or more second biodegradable superabsorbent polymer grades (bioSAP2). According to an embodiment, the top layer (L1) of the absorbent core comprises one or more first biodegradable superabsorbent polymer grades (bioSAP1) and the bottom layer (L2) comprises synthetic superabsorbent polymer grades (SAP2). According to an embodiment, the top layer (L1) of the absorbent core comprises one or more first biodegradable superabsorbent polymer grades and one or more second biodegradable superabsorbent polymer grades (bioSAP1) and the bottom layer (L2) comprises synthetic superabsorbent polymer grades (SAP2). According to an embodiment, the top layer (L1) of the absorbent core comprises one or more first biodegradable superabsorbent polymer grades (bioSAP1) and the bottom layer (L2) comprises does not comprise any superabsorbent polymer grades, only cellulosic fibers.

The first biodegradable superabsorbent polymer grades (bioSAP1) can have a color difference ($\Delta E$, C.I.E. L*scores, C.I.E. L*a*b* values) that is greater or lower than the color difference ($\Delta E$, C.I.E. L*scores, C.I.E. L*a*b* values) second superabsorbent polymer grades (SAP2) between a dry-state and wet-state.

For embodiments where the top layer (L1) of the absorbent core comprises one or more first biodegradable superabsorbent polymer grades (bioSAP1) and the bottom layer (L2) comprises one or more second biodegradable superabsorbent polymer grades (bioSAP2), the first biodegradable superabsorbent polymer grades (bioSAP1) can have an AUL that is greater than the AUL of second biodegradable superabsorbent polymer grades (bioSAP2), preferably the first biodegradable superabsorbent polymer grades (bioSAP1) have an AUL, as measured according to the test method herein, of greater than 15 g/g. Having such a higher AUL SAP on the top layer of the core limits rewet by providing resistance to squeeze-out of liquid upon application of forces whilst at the same time limiting the effects of gel-blocking. The lower layer may then have fast absorbing bioSAP that generally comes with poorer/lower AULs in order to promote fast absorption speeds and aid the draining effect mechanism supported by the cellulose fibers acting as liquid conduits towards the dispersed SAP.

In an embodiment, the cellulose fibers, whether conventional, TCF or ECF, are comprised at a level of at least 20% wt., preferably from 25% wt. to 40% wt., even more preferably from 28% wt. to 37%wt., by total weight of the absorbent material. Advantageously, cellulose fibers in these amounts allow for fast distribution of liquid across the core surface but if the amount is too high leads to poor rewet performance and/or excessive thickness and bulkiness of the core which reduces comfort of wear.

In an embodiment, and in particular preferably in further combination with the previous embodiment describing an absorbent core with two layers L1,L2 and with the above mentioned cellulose fiber content, the bioSAP Ratio (bioSAP1/bioSAP2) is from 0,3 to 3, preferably from 0,5 to less than 3, even more preferably from 0,7 to 2,5, even more preferably from 1 to 2, even more preferably from greater than 1 to less than 2; and/or wherein bioSAP1 is comprised at a level of from more than 25%wt to 75%wt, preferably from 30%wt to less than 75%wt, even more preferably from 35%wt to 70%wt, even more preferably from 40%wt to 65%wt, even more preferably from 41% to 64%, by total weight of the superabsorbent polymers (bioSAP1+bioSAP2). Advantageously, the bioSAP1 ratio and/or content has been found to synergistically work with the cellulose fibers/fluff content to limit sponge-like effects and/or squeeze-out of liquid following application of a load or force.

In an alternative, or complementary, embodiment the bioSAP Ratio (bioSAP1/bioSAP2) is less than 1, preferably from 0,2 to 0,9, more preferably from 0,3 to 0,85, even more preferably 0,4 to 0,8; and/or wherein bioSAP1 is comprised at a level of from more than 10%wt to 50%wt, preferably from 15%wt to less than 50%wt, even more preferably from 20%wt to 45%wt, even more preferably from 25%wt to 40%wt, even more preferably from 30%wt to 35%wt, by total weight of the superabsorbent polymer (bioSAP1+bioSAP2).

In an embodiment, the core wrap substrate (6) comprises an upper layer and a lower layer and the absorbent material is sandwiched therebetween with the upper layer positioned at a skin-facing (or body-facing) side of the absorbent material and the lower layer positioned at an opposite garment facing side of said absorbent material. The upper and lower layers are preferably joined together at one or more attachment areas positioned inboard of a perimeter of the absorbent core such to form one or more channels (10) substantially free of absorbent material as illustrated in FIG. 1. The channel(s) typically have a length extending in the longest dimension of the core that is from 10% to 95%, preferably from 15% to 90%, even more preferably from 20% to 85%, of the length of said longest dimension of the core. Exemplary channel geometries are illustrated in Fig. 4. The upper layers of the core wrap may be joined together at the attachment areas via one or more adhesives and/or one or more mechanical bonds (such as ultrasonic bonding, pressure bonding, thermal bonding, and combinations thereof). Advantageously, such channel structures further allow to reduce the overall amount of absorbent material used thus improving cost efficiencies and further improves liquid distribution speed across the core that in turn allows for further SAP combinations as will be discussed herein.

Typically the upper and lower core wrap layers 6 are, directly or indirectly, joined together at one or more positions inboard of the perimeter of the absorbent core and may be distinct to (or disconnected or inboardly spaced from) said perimeter to form channels. The upper and lower core wrap layers 6 can also come into contact with said perimeter, or at said perimeter or along said perimeter, to fully enclose or wrap the absorbent material. As exemplified in Fig. 4, the upper and lower core wrap layers are joined together at one or more attachment zones to form one or more channels (10), preferably a single channel, substantially free of absorbent material. Advantageously such channel structures aid fluid distribution along and across the core.

The absorbent articles 1 herein are preferably diapers or pants for babies or adults. In case of diapers preferred further components used in the art include: elastic ears or side panels for the fastening thereof, fastening systems comprising hook and loop and/or adhesive; leg cuffs; transversal front and back barriers or cuffs; acquisition distribution layers, wetness indicators etc. In case of pants preferred are 3-piece pants wherein front and back elasticized belts are joined via an absorbent insert comprising the topsheet, backsheet and absorbent core therebetween. The elasticized belts typically comprise a plurality of elastic strands or unitary elastic film. Further components in pants may be similar to those found in diapers such as leg cuffs; transversal front and back barriers or cuffs; acquisition distribution layers, wetness indicators etc.

In an embodiment, the absorbent article herein further comprises an acquisition distribution layer (7) positioned between the topsheet (2) and the core wrap substrate (6), preferably an upper layer thereof, and wherein the majority (typically being more than 50%, preferably more than 60%, even more preferably more than 70%, even more preferably more than 80%, even more preferably more than 80%, of the surface area of said acquisition distribution layer taken from a planar view formed by the longitudinal axis y and a transverse axis perpendicular thereto) of the surface of said acquisition distribution layer is in direct contact at least with said core wrap substrate; and wherein the acquisition distribution layer comprises, preferably consists of, natural fibers. For example, the acquisition distribution layer (7) can comprise a layer consisting essentially of viscose, such as an airlaid viscose layer.

As exemplified in Fig. 5, preferably the channel(s) have a shape forming at least one, preferably at least two, preferably from 2 to less than 10, even more preferably from 2 to 6, even more preferably from 2 to 5, typically only two, clusters ($C_s$) of absorbent material circumscribed by said attachment zones and preferably wherein the clusters ($C_s$) are spaced apart along a dimension parallel to the longitudinal axis (y), and even more preferably wherein the clusters ($C_s$) are connected by one or more attachment zones bridging between said clusters and extending substantially along said longitudinal axis (y). Advantageously, such clusters improve core stability of the multi-layer/multi-SAP grade cores especially upon wetting and especially in absence of any further layer (e.g. a further nonwoven or tissue) separating the two or more absorbent core layers leading to an optimal cost-effective solution that may avoid the incorporation of such further layers. However, having too many such clusters may result in a stiffening of the absorbent core upon saturation and swelling (typically as a result of wetting) of the absorbent material the optional upper limit of such clusters ensures to limit such effects for improved further comfort. In an embodiment, at least two clusters ($C_s$) of the one or more clusters have an area (on a plane corresponding to that formed by the longitudinal axis y and the width of the absorbent article perpendicular to said longitudinal axis y, i.e. as viewed from a planar view as exemplarily illustrated in Fig. 7) that is different in that said area of a first cluster is greater than said area of a second cluster, preferably wherein the first cluster is positioned closer to a front end of the diaper compared to the second cluster (as exemplarily shown in Fig. 7).

Absorbent cores comprised in absorbent articles of the present disclosure comprise specific SAP grades and/or types in specific layers of the multi-layer core construct.

The biodegradable superabsorbent polymers and synthetic superabsorbent polymers referred to herein are in the form of particles or granules.

AUL (Absorbency Under Load, 0,7 psi)

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0,7 psi is a Plexiglas cylinder 60 mm in internal diameter and 50 mm in height. Adhesively attached to its underside is a stainless steel sieve bottom having a mesh size of 36 $\mu$m. The measuring cell further includes a plastic plate having a diameter of 59 mm and a weight which can be placed in the measuring cell together with the plastic plate. The weight of the plastic plate and the weight together weigh 1345 g. AUL 0,7 psi is determined by determining the weight of the empty Plexiglas cylinder and of the plastic plate and recording it as WO. Then 0,900 +/- 0,005 g of water-absorbing polymer or material (particle size distribution 150 - 800 $\mu$m or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0,9% by weight sodium chloride solution is introduced for the surface of the liquid to be level with the filter plate surface without the surface of the filter plate being wetted. A round filter paper 90 mm in diameter and < 20 $\mu$m in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows:

$$AUL0,7psig/g=Wb-Wa/Wa-W0$$

AUL 0,3 psi and 0,5 psi are measured similarly at the appropriate lower pressure.

Absorbption speed (Vortex) measurement:

The vortex test measures the amount of time in seconds required for 2 grams of a superabsorbent material to close a vortex created by stirring 50 milliliters of saline solution at 600 revolutions per minute on a magnetic stir plate. The time it takes for the vortex to close (that the surface of the fluid becomes flat meaning that in the beginning, the centrifugal force that is caused by the rotation of the fluid creates a "coning-in" in the surface, but when the gelling of the SAP proceeds the viscosity of the fluid increases so that the depth of the indentation decreases until it's finally substantially flat) is an indication of the free swell absorbing rate of the superabsorbent material.

Equipment and materials:

1. Beaker, 100 ml.
2. Programmable magnetic stir plate, capable of providing 600 revolutions per minute.
3. Magnetic stir bar without rings, 7,9 mm × 32 mm, Teflon covered.
4. Stopwatch.
5. Balance, accurate to ± 0,01 g.
6. Saline solution, 0,9%.

7. Weighing paper.
8. Room with standard condition atmosphere: Temp = 23°C $\pm$ 1°C and Relative Humidity = 50% $\pm$ 2%.

Test procedure:

1. Measure 50 g $\pm$ 0,01 g of saline solution into the 100 ml beaker.
2. Place the magnetic stir bar into the beaker.
3. Program the magnetic stir plate to 600 revolutions per minute.
4. Place the beaker on the center of the magnetic stir plate such that the magnetic stir bar is activated. The bottom of the vortex should be near the top of the stir bar.
5. Weigh out 2 g $\pm$ 0,01 g of the superabsorbent material to be tested on weighing paper.
6. While the saline solution is being stirred, pour the superabsorbent material to be tested into the saline solution and start the stopwatch. The superabsorbent material to be tested should be added to the saline solution between the center of the vortex and the side of the beaker.
7. Stop the stopwatch when the surface of the saline solution becomes flat, and record the time.
8. The time, recorded in seconds, is reported as the Vortex Time.

The following SAP grades are used:

SAP1 - Ekotec EK-X52A (AUL 0,7 psi 20 g/g; absorption speed/Vortex 70 sec);

SAP2 - Sumitomo SA60N Typ II, sold under the Aqua Keep brand (AUL 0,7 psi 12 g/g; absorption speed/Vortex 34 sec).

Similar samples are prepared as above, with 12,3g of SAP1 and SAP2 but this time with 7g of cellulose fibers/fluff. At least 5 samples at each concentration are used in this example.

The front and the back regions of each sample of baby diaper described above, are clamped between two spacers to allow bowl shape formation. 210 mL tap water is poured into the formed bowl-shaped cavity on the body facing side of the diaper. The liquid is allowed to be taken up by the absorbent core for 30 seconds. After the 30 seconds are elapsed the diaper is squeezed for 5 seconds. While wringing the body facing side faces toward the outside, allowing the core to twist on itself, the squeezed water is collected in a receptacle and weighed and the "squeezed water" in g is recorded for each sample tested.

It is supposed that the present invention is not restricted to any form of realization described previously and that some modifications can be added to the presented example of fabrication without reappraisal of the appended claims and the embodiments can be combined. For example the absorbent article 1 can comprise a low-opacity topsheet 2, meaning a topsheet with a lower opacity value than the absorbent core or backsheet, and be free of any acquisition distribution layer 7, absorbent article 1 can comprise a low-opacity backsheet 3, an acquisition distribution layer 7 and comprise a upper core wrap 6a with a greater basis weight than the lower core wrap 6b.

The present disclosure also pertains to the use of biodegradable superabsorbent polymer in an absorbent article to measure the C.I.E L* scores of said absorbent article in a dry-state and a at least partially wetted state or wet-state to provide an indication of the state of said absorbent article selected from a dry-state, a partially wet-state or wet-state.

For example, conventional disposable absorbent articles can comprise a wetness indicator, said wetness indicators being visible from the exterior of the article and having the capacity to change appearance when contacted with body exudates, in particular urine. Usually wetness indicators are in the form of a hot-melt adhesive including one or more polymers to provide cohesive strength, a resin or analogous material (tackifier) to provide adhesive strength; and optional waxes, plasticizers or other materials to modify viscosity, and/or other additives including, but not limited to, antioxidants or other stabilizers these components comprises several chemical products and typically several petroleum-based polymers which further present issues such as sustainability. The use of biodegradable superabsorbent polymer in an absorbent article to measure the C.I.E L* scores of said absorbent article in a dry-state and a at least partially wetted state or wet-state to detect a wet and/or dry-state of absorbent article remove the need for conventional wetness indicators thereby lowering the presence of petroleum derived polymers.

**Claims**

**1.** Absorbent article (1) comprising:

- a liquid permeable topsheet (2),

- a liquid impermeable backsheet (3), and
- an absorbent core (4) positioned between said topsheet (2) and backsheet (3),

wherein the absorbent core (4) comprises an absorbent material (5), said absorbent material comprising a mixture, or blend, of cellulose fibers (5a) and superabsorbent material (5b), **characterized in that** the superabsorbent material (5b) comprises biodegradable superabsorbent material (bioSAP) that has a first color in dry state and a second color in wet state, and **in that** first color and the second color has a CIELab color space difference ($\Delta E$) of at least 2 as measured according to the test method herein.

2. Absorbent article (1) according to claim 1, the biodegradable superabsorbent material (bioSAP) has a $\Delta L^*$ greater than 5 between a dry-state and a wet-state, preferably greater than 10.

3. Absorbent article (1) according to claim 1 or 2, wherein the biodegradable superabsorbent material (bioSAP) comprises carbohydrate fermentation-derived polymers.

4. Absorbent article (1) according to claim 1 or 2, wherein the biodegradable superabsorbent material (bioSAP) comprise polysaccharides-derived polymers, preferably having crosslinked polysaccharides.

5. Absorbent article (1) according to any of the preceding claims, wherein the $\Delta E$ between the dry-state and the wet-state is greater than 10, preferably greater than 20.

6. Absorbent article (1) according to any of the preceding claims, wherein the absorbent core (4) comprises a first and a second layer (L1,L2), said first and second layer (L1,L2) containing different grade and/or concentration of biodegradable superabsorbent material (bioSAP).

7. Absorbent article (1) according to claim 6, wherein said first layer (L1) contains carbohydrate fermentation-derived polymers and second layer (L2) contains polysaccharides-derived polymers.

8. Absorbent article (1) according to claim 7, wherein said first layer (L1) is arranged proximal to the topsheet (2) and said second layer (L2) proximal to the backsheet (3).

9. Absorbent article (1) according to claim 6, wherein both first layer (L1) and second layer (L2) contains carbohydrate fermentation-derived polymers and polysaccharides-derived polymers.

10. Absorbent article (1) according to any of the preceding claims, wherein said color space difference ($\Delta E$) between the dry-state and the wet-state is greater than 10, preferably greater than 20.

11. Absorbent article (1) according to claim 6 and following, wherein said color space difference ($\Delta E$) between a dry-state and a wet-state is greater for the first layer (L1) than for the second layer (L2).

12. Absorbent article according to any of the preceding claims, wherein the cellulose fibers are comprised at a level of at least 20%wt, preferably from 25%wt to 40%wt, even more preferably from 29%wt to 37%wt, by total weight of the absorbent material.

13. Absorbent article according to any of the preceding claims, wherein the absorbent material (5) is wrapped in a core wrap substrate (6), said core wrap substrate (6) comprising upper and lower layers (6a,6b) that are joined together by one or more adhesives and/or by one or more mechanical bonds selected from the group consisting of ultrasonic bonds, thermal bonds, pressure bonds, and combinations thereof, preferably said layers are joined together at one or more attachment zones to form one or more channels (10), preferably a single channel, substantially free of absorbent material.

14. Absorbent article according to any of the preceding claims, wherein the cellulose fibers (5a) are selected from unbleached pulp or totally chlorine free (TCF) or elemental chlorine free (ECF) cellulose fibers or any combination thereof.

15. Use of biodegradable superabsorbent polymer (bioSAP) in an absorbent article to provide a change of color between a dry-state and a at least partially wet-state, preferably to provide an indication of the state of said absorbent article selected from a dry-state, a partially wet-state or wet-state.

**FIG. 1**

**FIG. 2**

**FIG. 3**

**FIG. 4**

FIG. 5

**FIG. 6**

Europäisches
Patentamt

European
Patent Office

Office européen
des brevets

# EUROPEAN SEARCH REPORT

**Application Number**

EP 24 18 5078

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/054782 A1 (CHAN RYAN NICHOLAS [US] ET AL) 20 February 2020 (2020-02-20) * The whole document, in particular Figs. 12-14; par. [0019], [0046]-[0051], [0058]-[0067], [0090], [0100], [0153]-[0171], [0186]-[0191]; examples 10-12; claims * | 1-15 | INV. A61F13/53 A61F13/42 A61L15/28 A61L15/60 |
| X | US 2017/021051 A1 (RICHARDS NICOLE M [US] ET AL) 26 January 2017 (2017-01-26) * The whole document, in particular Figs., par. [0057], [0066], [0082]-[0093], [0165]-[0176] * | 1-13,15 | |
| X,D | US 2017/224540 A1 (LI CLIVE [US] ET AL) 10 August 2017 (2017-08-10) * The whole document, in particular Fig. 1; par. [0032]-[0036], [0065], [0066] * | 1-15 | |

**TECHNICAL FIELDS SEARCHED (IPC)**

A61F
A61L

The present search report has been drawn up for all claims

| Place of search | Date of completion of the search | Examiner |
|---|---|---|
| Munich | 28 November 2024 | Schmitt-Humbert, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 5078

This annex lists the patent family members relating to the patent documents cited in the above-mentioned European search report.
The members are as contained in the European Patent Office EDP file on
The European Patent Office is in no way liable for these particulars which are merely given for the purpose of information.

28-11-2024

| Patent document cited in search report | Publication date | Patent family member(s) | Publication date |
|---|---|---|---|
| US 2020054782 A1 | 20-02-2020 | US 2020054782 A1 | 20-02-2020 |
| | | US 2021244844 A1 | 12-08-2021 |
| US 2017021051 A1 | 26-01-2017 | CN 108135760 A | 08-06-2018 |
| | | EP 3324910 A1 | 30-05-2018 |
| | | HK 1254610 A1 | 26-07-2019 |
| | | JP 6805249 B2 | 23-12-2020 |
| | | JP 2018520837 A | 02-08-2018 |
| | | KR 20180041117 A | 23-04-2018 |
| | | US 2017021051 A1 | 26-01-2017 |
| | | US 2020316245 A1 | 08-10-2020 |
| | | WO 2017015477 A1 | 26-01-2017 |
| US 2017224540 A1 | 10-08-2017 | US 2017224540 A1 | 10-08-2017 |
| | | WO 2016023016 A1 | 11-02-2016 |

EPO FORM P0459

For more details about this annex : see Official Journal of the European Patent Office, No. 12/82

**EP 4 606 361 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20230058841 A
- EP 4158049 A
- WO 2019118987 A
- US 20170224540 A
- WO 2017061166 A
- US 3485706 A